# EUROPEAN PATENT APPLICATION

(11) **EP 4 272 653 A1**
(43) Date of publication of application: **08.11.2023**
(21) Application number: 23170405.7
(22) Date of filing: 27.04.2023
(51) Int. Cl.: A61B 8/00

(54) **A ROBOTIC SYSTEM FOR PERFORMING AN ULTRASOUND SCAN**

(30) Priority: 04.05.2022 DK PA202270235
(71) Applicant: Ropca ApS, 5230 Odense M (DK)
(72) Inventor: JØRGENSEN, Johannes Chemnitz Albinus, 5210 Odense NV (DK); SAVARIMUTHU, Thiusius Rajeeth, 5250 Odense SV (DK)
(74) Representative: AWA Denmark A/S

(57) **Abstract**

Disclosed is a robotic system for performing an ultrasound scan on a body part of a patient using an ultrasound probe. The robotic system comprises a movable support structure for supporting the body part; a positioning device configured to move the movable support structure to align the body part with the ultrasound probe to obtain an ultrasound scan of the body part supported by the movable support structure; and a control unit configured to generate a first movement instruction for the positioning device for moving the movable support structure to obtain an ultrasound image of the body part. Further disclosed is an ultrasound scanner comprising the robotic system.

## Description

### Technical field

The present invention relates to a robotic system for performing an ultrasound scan on a body part of a patient.

### Background

Ultrasound tools are widely employed in the medical world, for example for detecting or diagnosing injuries on body parts, such as hand or feet, or for diagnosing diseases, such as rheumatism or arthritis, in body parts.

Commonly such ultrasound scans are carried out by trained medical personnel specialised or trained in the field of ultrasound. Often the medical personnel operate the ultrasound probe to locate this across the area of interest on the body part. This may, however, be a time-consuming process and with the limited amount of available skilled medical personnel, this may lead to long wait time for scans at hospitals or clinics, which again can lead to a worsening of the injury or disease of the patient. Furthermore, even the skilled personnel may have difficulties in performing consistent and efficient ultrasound scans.

Robotic ultrasound systems have been developed to overcome some of these problems, as these may free up time for the medical personnel by performing certain ultrasound scans semi-automatically or fully automatically. Such robotic ultrasound systems typically have a robotic arm, which controls and moves the ultrasound probe to the area of interest on the body part of the patient.

US 2017/0181725 A1 discloses an exemplary ultrasound imaging system including a scanning assembly, a three-dimensional (3D) image acquisition device, and a controller. The scanning assembly is configured to receive a hand or foot and includes a transducer array and an acoustic coupling fluid. The 3D image acquisition device is configured for obtaining a 3D image of the hand or foot. The controller is configured for automatically adjusting direction or orientation of the transducer array with respect to the hand or foot based on the 3D image of the hand or foot.

EP 2 514 366 A1 discloses an exemplary automatic ultrasonic scanning system and a scanning method. The automatic ultrasonic scanning system includes a multi-axis robot arm, an ultrasonic scan head disposed on the multi-axis robot arm, a control circuit for controlling the multi-axis robot arm, three-dimensional image capturing apparatus and a computer.

These robotic ultrasound systems, however, still suffer from drawbacks. Typically, such robotic systems require a complex robotic arm with several degrees of freedom in operation to allow the ultrasound system to sufficiently scan a body part of patient. Such robotic arms may furthermore be large and/or bulky looking which may frighten a patient, who is having an ultrasound scan performed.

### Summary

It is therefore an object to provide a robotic system, which overcomes or at least alleviates the problems of the prior art.

A first aspect of the present disclosure relates to a robotic system for performing an ultrasound scan on a body part of a patient using an ultrasound probe, the robotic system comprising:
a movable support structure for supporting the body part;
a positioning device configured to move the movable support structure to align the body part with the ultrasound probe to obtain an ultrasound scan of the body part supported by the movable support structure; and
a control unit configured to:
   generate a first movement instruction for the positioning device for moving the movable support structure to obtain an ultrasound image of the body part.

It has been realised that by moving a movable support structure to align the body part with the ultrasound probe, a simpler robotic system is provided. For instance, by moving the positioning device and thus the body part to be aligned with the probe, fewer degrees of freedom may be needed for a robotic arm holding the probe, which in turn allows for a less complex and lighter robotic arm. Thereby, the design and manufacturing efforts and costs as well as the service requirements for the robotic arm be reduced. In some examples, the probe may not need to be installed on a robotic arm as such but may be kept stationary, thus removing the need for the complex and heavy robotic arm holding the probe.

Furthermore, the movable support structure further allows for the probe to be arranged in a position, where it is hidden from a user, such as underneath the support structure when seen from a user. Where the robotic system is, for instance, integrated in a movable ultrasound system, this allows for a more compact ultrasound system. Additionally, by allowing the probe and robotic arm, if any, to be hidden from the patient, no visible robotic arms are present, thereby reducing the risk of frightening the patient.

Having the movable support structure and the positioning device configured to move the movable support structure to align the body part with the ultrasound probe can assure that the body part to be scanned is always accessible by the probe.

By the body part being aligned with the ultrasound probe may herein be understood that the body part is arranged at an imaging position, in which an ultrasound image is obtainable by the ultrasound probe.

It will furthermore be appreciated that by ultrasound scan may throughout this text be understood obtaining one or more ultrasound images of the body part by means of the ultrasound probe.

The movable support structure may be configured to support the body part. By supporting the body part may herein be understood that the movable support structure may be configured to let the body part remain at a certain position when no force is applied by the body part. As an example, the movable support structure may be configured to support the body part by allowing the body part to rest on it, to suspend the body part, and/or to hold the body part.

The robotic system may further comprise additional elements and/or may be integrated into an ultrasound system or ultrasound scanner, such as a movable ultrasound system or movable ultrasound scanner. Alternatively or additionally, the robotic system may be configured to be connected to a, potentially movable, ultrasound system or ultrasound scanner.

Alternatively or additionally, at least the support structure and positioning device of the robotic system may be arranged on a structure. The robotic system may comprise an enclosure, which may enclose one or more of the positioning device and at least a portion of the movable support structure. Where an enclosure is provided, the enclosure may comprise a portion and/or a surface for supporting a further part of the body of the patient. For instance, where the body part to be scanned is part of a hand, the enclosure may comprise support for a wrist or a lower arm of the patient.

In some embodiments, the control unit may comprise a processing unit configured to generate a first movement instruction for the positioning device for moving the movable support structure to obtain an ultrasound image of the body part. The control unit may further comprise a potentially non-transitory memory comprising instructions, which, when executed, cause the processing unit to generate the first movement instructions. Alternatively or additionally, the instructions may be stored on a non-transitory computer-readable storage medium.

It will be appreciated that, in the preceding and the following, any steps described as being performed by the control unit may be performed by the processing unit and/or that the memory may comprise instructions which, when executed, cause the processing unit to perform said steps.

The control unit or processing unit may comprise or be any one of a micro controller unit (MCU), a central processing unit (CPU), a field-programmable gate array (FPGA), Programmable Logic Array (PLA), a Digital Signal Processor (DSP), or any combination thereof. The control unit or processing unit may be communicatively connected to the positioning device and/or may be configured to control operation of the positioning device.

Alternatively or additionally, the control unit may be communicatively connected to the ultrasound probe and/or may be configured to control operation of the ultrasound probe, such as cause the ultrasound probe to obtain an ultrasound scan and/or control emission of ultrasound from the ultrasound probe.

In some embodiments, the control unit is further configured to obtain an ultrasound scan of the body part.

Alternatively or additionally, the robotic system may further comprise an ultrasound processing unit for performing one or more of obtaining an ultrasound scan from the ultrasound probe, receive data from the ultrasound probe indicative of detected ultrasound waves, and/or generate ultrasound images based on data indicative of detected ultrasound waves received from the ultrasound probe. The ultrasound processing unit may be operationally connected to the control unit.

In some embodiments, the control unit may generate a first movement instruction based on a desired alignment of the body part with the ultrasound probe. Alternatively or additionally, the control unit may further be configured to determine a position of the body part. The control unit may be configured to determine a motion path for the movable surface to align the body part and generate the first movement instruction based on the motion path.

The control unit may be configured to determine a first pre-scan position of the body part and determine first movement instructions, potentially based on a motion path, for the positioning device for moving the movable surface to a scan position, in which the body part is aligned with the ultrasound probe.

Throughout this disclosure, the first and any further movement instruction may cause the positioning device to move the movable support structure to align the body part with the ultrasound probe.

The control unit may be configured to determine the position of the body part using a sensor, such as a camera or LIDAR sensor, and/or based on knowledge about the movable support structure and/or a surface of the movable support structure. The knowledge about the movable support structure may comprise knowledge about one or more marks of the movable structure and/or their positions. For instance, where the movable support structure comprises an indentation to receive the body part, potentially shaped to receive the body part, the control unit may generate the first movement instructions based at least in part on knowledge about the indentation and/or its position.

Alternatively or additionally, the control unit may be configured to, potentially repeatably, obtain a first ultrasound image and determine if the body part is aligned with the ultrasound probe. Where the control unit determines that the body part is not aligned with the ultrasound probe, the control may generate a first or a further movement instruction for the positioning device for moving the movable support structure to obtain a further ultrasound image of the body part. The determination whether the body part is aligned or not may be performed by evaluating the first ultrasound image.

Evaluating the first ultrasound image may comprise assigning a score to the first ultrasound image. Evaluating the first ultrasound image may comprise determining anatomical features in the first ultrasound image, e.g. a location of a joint in the first ultrasound image may be determined. Evaluating the first ultrasound image may comprise determining an image quality of the first ultrasound image. Evaluating the first ultrasound image may comprise determining an instruction for the positioning device to obtain a higher quality second ultrasound image.

The machine learning data architecture may be trained by evaluating a training data set comprising plurality of training ultrasound images. The plurality oftraining ultrasound images may be ultrasound images obtained by medical professionals. The plurality of training ultrasound images may be ultrasound images previously obtained by the robotic system. Each of the training ultrasound images may have a score attached to them to indicate the quality of the training ultrasound images indicating the quality of the ultrasound image. The score may be assigned by a medical professional, i.e. the machine learning data architecture may be a supervised learning model. The quality may indicate the ability of the ultrasound image for being used for diagnostic purposes. The medical professional may use his / her experience when assigning scores. The score may be a binary score e.g. bad / good, or a score on a scale e.g. from 0 to 100. The machine learning data architecture may be trained for different anatomical features, e.g. for evaluating different joints in hands and feet. Thus, for each joint the machine learning data architecture may have been provided with a specific training data set. The machine learning data architecture may be an artificial neural network such as a deep structured learning architecture. If the robotic system is to be used for scanning joints, the machine learning data architecture may be trained to identify and evaluate an image quality of a joint capsule and bones surrounding the joint capsule.

In some embodiments, the ultrasound probe is fixed with respect to and/or integrated in the robotic system. The ultrasound probe may be arranged at a fixed position and/or may be kept stationary relative to other parts of the robotic system, such as the positioning device, the movable support structure, and/or the control unit.

The ultrasound probe may be arranged on and/or held by a structure. The structure may be a movable structure configured to move the ultrasound probe relative to the movable support structure and/or the positioning device. The ultrasound probe may, e.g. by means of the structure, be movable translationally in one or more directions and/or rotationally about one or more rotational axes. The robotic arm may be operationally connected to a positioning device configured to move the movable structure so as to move the probe to an ultrasound scan. The positioning device may be the positioning device configured to move the movable support structure or a second positioning device.

In an example, the probe may be arranged on a movable structure configured to move the ultrasound probe in one direction, such as a vertical or substantially vertical direction. The control unit may, in this example, generate first movement instructions for the positioning device to move the movable support structure to a position, in which the body part is aligned with the ultrasound probe, so that a movement of the ultrasound probe in the direction allows the probe to reach an imaging position, in which an ultrasound image of the body part. The first movement instruction may, in this example, further comprise movement instructions for the positioning device or for a second positioning device for moving the ultrasound probe to the imaging position by means of the movable structure. Alternatively, the control unit may, in this example, generate a second movement instruction for the positioning device or for a second positioning device for moving the ultrasound probe to the imaging position by means of the movable structure.

In some embodiments, the movable structure may be an arm. In some embodiments, the arm is configured to move the probe translationally in one or more directions and/or rotationally around one or more axes in response to a movement instruction, such as a first or second movement instruction.

In some embodiments, the body part is at least a part of a hand or at least a part of a foot. If the body part is at least a part of a hand, the body part may also constitute at least a part of an arm. Similarly, if the body part is at least a part of a foot, the body part may also constitute at least a part of a leg. In one embodiment the body part is at least a part of a leg or at least a part of an arm.

For instance, the body part may be a finger joint, a wrist, or any other joint in a hand or in a foot. The body part may, alternatively or additionally, be any part of a body of a patient, such as a leg, a knee, a shoulder, an elbow, an arm, a torso, a neck, etc. In some embodiments, the body part may be a joint of a body.

In some embodiments, the movable support structure comprises a substantially planar surface or a planar surface. The planar surface may facilitate that the joint angles of the body part to be scanned are substantially zero which may be an advantage during some scanning procedures.

In another embodiment, the support structure comprises a substantially curved surface or a curved surface. The curved surface or the substantially curved surface may be a dome-shaped surface.

The curved surface may create an ergonomically comfortable position for the body part. It may facilitate a more secure placement of the body part on the support structure such that displacement of the body part on the support structure is minimized during movement of the support structure.

Where the movable support structure comprises a curved or substantially curved surface, the movable support structure may be spherical or substantially spherical, such as hemi-sphere shaped, or may be spheroid or substantially spheroid, such as a hemi-spheroid.

In another embodiment the movable support structure may comprise a partly planar and partly curved surface. For example, the surface in contact with the body part may be planer, while a part of the surrounding surface of the movable support structure may be curved. In another embodiment the surface in contact with the body part may be curved while the remaining support structure may be partly or fully curved.

In some embodiments, the movable support structure has one or more indentations configured to conform to the body part. The indentations may serve as support for the body part and make the scanning procedure more comfortable for the user. The indentations may be in a planar surface or in a curved surface. It is to be understood that the surface can be planar in a general sense with indentations in that planar surface. Similarly, the surface may take an overall curved shape with indentations in the curved surface to accommodate the body part. The indentations may provide support for the body part to be scanned. The indentations may be substantially formed as to conform to the body part. The indentations may have a depth smaller than the thickness of the body part such that the body part partly sticks out from the indentations. The indentations may in another embodiment have a depth greater than the thickness of the body part. The indentations may in yet another embodiment have a depth substantially corresponding to the thickness of the body part. The at least one indentation may have varying depths.

In some embodiments, the movable support structure is movable translationally in one or more directions and/or rotationally around one or more axes.

The movable support structure may have a docking position in which the body part is placed on the movable support structure. The docking position may be at a distance away from the probe which must be traversed to align with the probe. The movable support structure may traverse this distance to align the body part with the ultrasound probe. This distance may be traversed by translational movement, rotational movement, or a combination thereof of the movable support structure.

The movable support structure is movable relative to the ultrasound probe such that a position, in which an ultrasound image of the body part can be obtained, is achieved.

The movable support structure is moved by a positioning device. The positioning device is controlled by a control unit. The positioning may comprise actuators. The positioning device may comprise gears. The positioning device may comprise pistons. The positioning device may comprise at least one belt, each belt joined to at least one wheel to displace the at least one belt. The positioning device may be a hydraulic system. The positioning device may an AC motor. The positioning device may be a DC motor. The positioning device may comprise a combination thereof. The positioning device may be realised by any computer controlled actuating devices that result in translational and/or rotational movement.

In some embodiments, the control unit is configured to generate a second movement instruction for moving the ultrasound probe towards the body part by means of a positioning device configured to move the ultrasound probe to obtain an ultrasound scan of the body part.

The positioning device configured to move the ultrasound probe may be the same positioning device configured to move the movable support structure.

The positioning device configured to move the ultrasound probe may be a separate positioning device from the one configured to move the movable support structure, such as a second positioning device.

The positioning device configured to move the ultrasound probe may either bring the ultrasound probe towards or away from the body part. This may provide a way to adjust the focus of the ultrasound image. This may further facilitate that the movable support structure can move more freely without obstruction from the ultrasound probe prior to the ultrasound probe being moved towards the body part.

The positioning device may be electrically coupled to the control unit.

The control unit may be configured to generate the second movement instruction in a similar manner to the generation of the first movement instruction. For instance, the control unit may be configured to generate the second movement instruction in response to a determination that the positioning device has moved the movable support structure, potentially according to the first movement instruction, to align the body part with the probe. Correspondingly, the control unit may further be configured to determine that the positioning device has moved the movable support structure, potentially according to the first movement instruction, to align the body part with the probe.

In some embodiments, the movable support structure is arranged on the same side of the body part as the ultrasound probe.

The ultrasound probe may be placed substantially behind the movable support structure relative to the body part. The ultrasound probe may in this way be partly or completely hidden from the user.

In such an embodiment, the movable support structure may be configured to provide an acoustic impedance match between the body part and the ultrasound probe.

By the movable support structure being configured to provide an acoustic impedance match may herein be understood that the movable support structure has an acoustic impedance that allows an ultrasound image of the body part to be obtained through the movable support structure. The specific acoustic impedance of the support structure may be substantially the same or the same as a specific acoustic impedance of soft tissue. Throughout this disclosure, acoustic impedance refers to the acoustic impedance at least in the ultrasound range, such as in the range from 2 MHz to 15 MHz.

Alternatively or additionally, the movable support structure may act as a matching layer for the ultrasound probe. The acoustic impedance of the movable support structure may be at a value between the acoustic impedance of an ultrasound crystal of the ultrasound probe and the acoustic impedance of soft tissue.

In some embodiments, at least a portion of the movable support surface may be configured to provide an acoustic impedance match between the body part and the ultrasound probe. At least a portion of the movable support structure may be made of ballistic gel. The ballistic gel may be a material having the same density and/or structure as human tissue.

In another embodiment, the movable support structure has at least one aperture allowing at least a portion of the ultrasound probe to pass through the at least one aperture. The apertures may allow the ultrasound probe to come in contact with the body part. In another embodiment the apertures may be smaller than the ultrasound probe. The apertures may be filled with ultrasound gel such that an ultrasound scan can be obtained without passing through the at least one aperture.

In some embodiments, the movable support structure comprises fixating means for maintaining a fixed position of the body part on the movable support structure during movement of the moveable support structure.

The support structure may comprise holes in the support structure upon which suction can be provided such that the body part can be fixed by means of vacuum.

The support structure may in another embodiment comprise a magnetic glove wherein the body part may be placed. By glove is meant a harness that fits the body part. The magnetic glove may be fixed to the support structure.

In another embodiment the support structure may comprise memory foam in which the body part is placed. The memory foam may upon adaptation to the body part act as support.

The support structure may comprise a belt strap.

A second aspect of the present disclosure relates to an ultrasound scanner comprising the robotic system according to the first aspect and an ultrasound probe.

In one embodiment the ultrasound scanner may comprise a display unit and/or a user input device.

The display unit may be or may comprise a flatscreen display, such as a Liquid Crystal Display (LCD), a Light Emitting Diode (LED) backlit display, a Plasma display or any combination thereof. The display unit may be configured to display instructions to a patient.

The user input device may be or may comprise a keyboard, such as a QWERTY keyboard or a regional equivalent or a numeric keyboard, a pointer device, such as a mouse or touchpad, and/or a touch display.

In some embodiments, the display unit may be configured to display one or more instructions and/or questions to a user, to which a user may respond using the user input device.

It is noted that the invention relates to all possible combinations of features recited in the claims. Other objectives, features, and advantages of the present inventive concept will appear from the following detailed disclosure, from the attached claims as well as from the drawings. A feature described in relation to one of the aspects may also be incorporated in the other aspect, and the advantage of the feature is applicable to all aspects in which it is incorporated.

A person skilled in the art will appreciate that any one or more of the above aspects of this disclosure and embodiments thereof may be combined with any one or more of the other aspects of the disclosure and embodiments thereof.

### Brief Description of Drawings

In the following description, embodiments of the robotic system and ultrasound scanner comprising the robotic system will be described with based on nonlimiting exemplary embodiments and with reference to the drawings, of which
FIG. 1 shows a block diagram of a robotic system according to an embodiment of the present disclosure,
FIG. 2 shows a schematic diagram of the robotic system shown in the block diagram in FIG. 1,
FIG. 3 shows a schematic diagram of a robotic system according to an embodiment of the present disclosure,
FIG. 4 shows a schematic diagram of a robotic system according to an embodiment of the present disclosure,
FIG. 5 shows a schematic diagram of a robotic system according to an embodiment of the present disclosure,
FIG. 6 shows a schematic diagram of a robotic system according to an embodiment of the present disclosure,
FIG. 7 shows a schematic diagram of a robotic system according to an embodiment of the present disclosure,
FIG. 8 shows a schematic perspective view of an ultrasound scanner comprising the robotic system shown in the block diagram shown in FIG. 1,
FIG. 9 shows a schematic perspective view of an ultrasound scanner comprising the robotic system shown in the block diagram shown in FIG. 1,
FIG. 10 shows a schematic perspective view of an ultrasound scanner comprising the robotic system shown in the schematic diagram shown in FIG. 4, and
FIG. 11 shows a schematic perspective view of an ultrasound scanner comprising the robotic system shown in the block diagram shown in FIG. 7.

Similar reference numerals are used for similar elements across the various embodiments and figures described herein.

### Detailed Description

The ultrasound scanner and robotic system according to the present disclosure will now be described more fully hereinafter with reference to the accompanying drawings, in which embodiments of the ultrasound scanner and robotic system according to the present disclosure are shown. The ultrasound scanner and robotic system may, however, be embodied in many different forms within the scope of the appended claims and should not be construed as limited to the embodiments set forth herein; rather, these embodiments are provided for thoroughness and completeness.

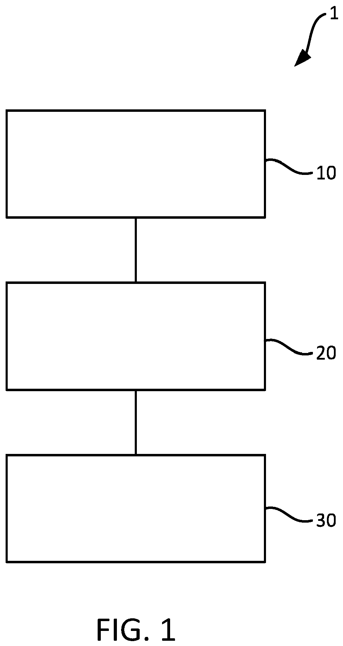

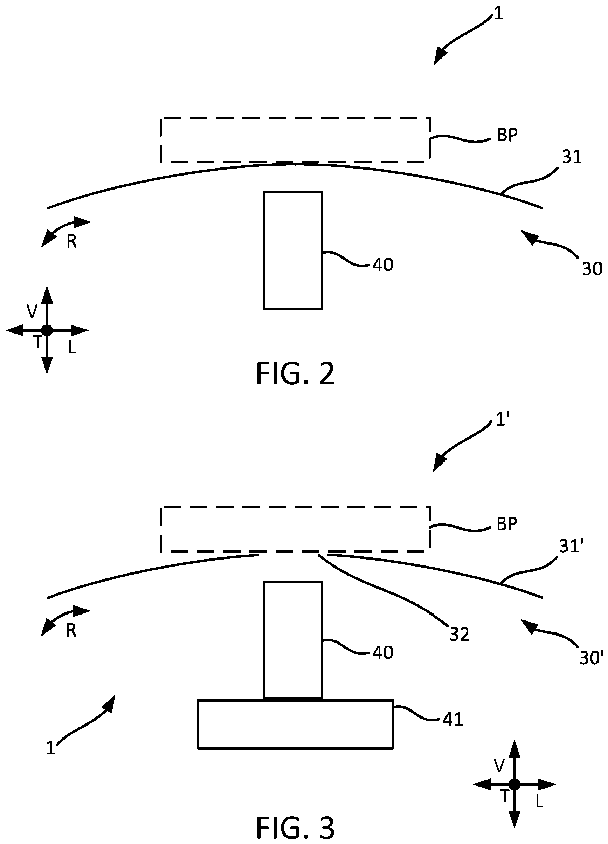

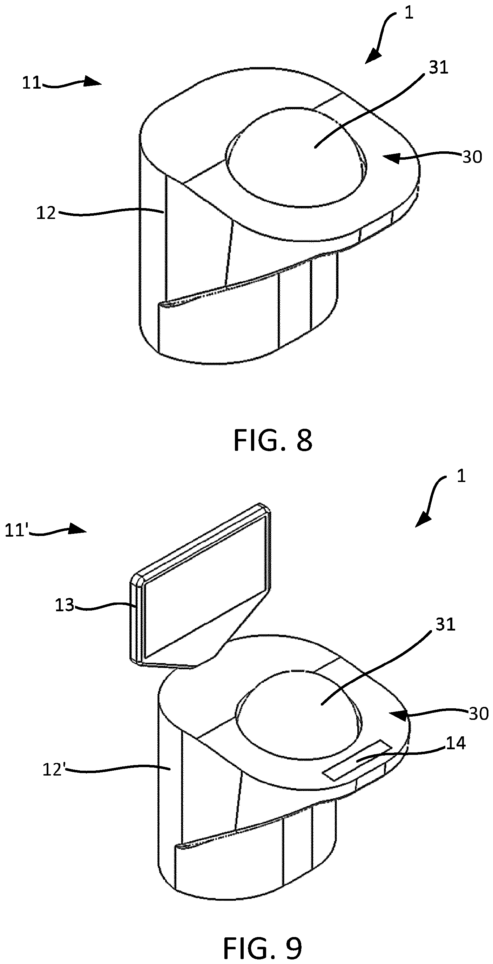

Referring initially to FIG. 1, FIG. 1 shows a block diagram of a robotic system 1 according to an embodiment of the present disclosure. FIG. 2 shows a schematic diagram of the robotic system 1 as well as a body part BP and an ultrasound probe 40, and FIG. 8 shows a schematic perspective view of an ultrasound scanner 11 comprising the robotic system 1.

The robotic system 1 is a robotic system for performing an ultrasound scan on a body part BP of a patient using an ultrasound probe 40, the robotic system 1 comprising a movable support structure 30 for supporting the body part BP. The robotic system 1 further comprises a positioning device 20 configured to move the movable support structure 30 to align the body part BP with the ultrasound probe 40 to obtain an ultrasound scan of the body part BP supported by the movable support structure 30. The robotic system 1 further comprises a control unit 10 configured to generate a first movement instruction for the positioning device 20 for moving the movable support structure 30 to obtain an ultrasound image of the body part BP.

The control unit 10 is operably connected to the positioning device 20. The control unit 10 may be electrically connected, such as wired or wirelessly connected to the positioning device 20. The positioning device 20 is operably connected to the movable support structure 30, such as mechanically connected.

As shown in FIG. 2, the movable support structure 30 comprises a surface 31. The surface 31 is a support surface configured to provide support for the body part BP. The surface 31 is a curved surface. The movable support structure 30 may be hemisphere-shaped and the surface 31 may be shaped as a surface of a hemisphere.

The movable support structure 30 is rotatable in a rotation direction R about a rotational axis (not shown). The positioning device 20 is configured to rotate the movable support structure in the rotation direction R.

In some embodiments, the rotation direction R is a first rotation direction and the rotational axis is a first rotational axis. The movable support structure 30 may further be configured to rotate in a second rotation direction around a second rotational axis, potentially perpendicular to the first rotational axis. The movable support structure may further be rotatable in a third rotation direction around a third rotational axis, potentially perpendicular to the first and second rotational axis.

In the present embodiment, the movable support structure is rotatable. In some embodiments, the movable support structure 30 may alternatively or additionally be movable translationally in one or more of a first direction, illustrated and interchangeably described as longitudinal direction L, a second direction, illustrated and interchangeably described as vertical direction V, which is substantially orthogonal to the longitudinal direction L, and a third direction, illustrated and interchangeably described as transversal direction T, which is substantially orthogonal to the longitudinal direction L and the vertical direction V.

As shown in FIGs. 2 and 8, the ultrasound probe 40 and the movable support structure 30 are arranged on the same side of the body part BP. The ultrasound probe 40 is underneath the movable support structure 30 when seen from the perspective of a user. Correspondingly, ultrasound probe 40 is hidden from a user underneath the movable support structure 30 as shown in FIG. 8.

The ultrasound probe 40 is configured to be stationary with respect to movable support structure 30. In other embodiments, the ultrasound probe 40 may be configured to be movable, such as rotational in a first R, second, and/or third rotational direction, and/or translationally movable in one or more of the longitudinal L, vertical V, and/or transversal directions T.

The movable support structure 30 has an acoustic impedance for ultrasound, which is substantially the same or the same as an acoustic impedance for ultrasound of skin of the body part BP. In other embodiments, the movable support structure may have a different acoustic impedance, such as a higher acoustic impedance, to match an acoustic impedance between an ultrasound transducer of the ultrasound probe 40 and the body part BP. The movable support structure 30 is made from a material, which is dimensionally stable whilst providing the acoustic impedance, so that the ultrasound probe 40 can obtain an ultrasound image of body part BP through the movable support structure 30 and its surface.

In FIG. 8, an ultrasound scanner 11 comprising the robotic system 1 and the ultrasound probe 40 is shown.

The ultrasound scanner 11 comprises a housing 12. The housing 12 provides an enclosure and houses control unit 10 and positioning device 20 of the robotic system 1 and the ultrasound probe 40. The housing 12 may be made from any suitable material, as will be well-known to the skilled person within the field of ultrasound scanners. For instance, the housing may be made from a polymer. Alternatively or additionally, the housing may comprise one or more of a polymer, a metal, and/or glass fibres.

In other embodiments, the control unit 10 may be arranged outside of the housing 12.

The ultrasound scanner 11 may be a stationary ultrasound scanner, i.e. an ultrasound scanner configured to be fastened to a floor, or may be a movable ultrasound scanner. Where the ultrasound scanner 11 is a movable ultrasound scanner, the housing may comprise and/or be arranged on one or more wheels, allowing personnel to move the ultrasound scanner.

FIG. 9 shows a schematic perspective view of an ultrasound scanner 11' comprising the robotic system 1.

The ultrasound scanner 11' differs from the ultrasound scanner 11 shown in FIG. 8 in that the ultrasound scanner 11' further comprises a display unit 13 and a user input device 14. The user input device 14 is a keyboard but may in other embodiments be or comprise one or more of a mouse, a touchpad, a touch display, or the like. The display unit 13 is a liquid crystal display.

Correspondingly, housing 12' differs from housing 12 in that the housing 12' comprises recesses for display unit 13 and user input device 14. In other embodiments, display unit 13 and/or user input device 14 may be arranged on housing 12.

It will, thus, be appreciated that any feature described with respect to ultrasound scanner 11 and housing 12 may apply to the ultrasound scanner 11' and the housing 12', respectively.

FIG. 3 shows a schematic diagram of a robotic system 1' according to an embodiment of the present disclosure.

The robotic system 1', similar to robotic system 1, comprises a control unit 10 and a positioning device 20.

The robotic system 1' comprises movable support structure 30' having a surface 31'. The surface 31' is configured to provide support to the body part BP. Similar to robotic system 1, the movable support structure 30' and the ultrasound probe 40 is arranged on the same side of the body part BP.

The movable support structure 30' and surface 31' differs from movable support structure 30 and surface 31, respectively, in that an aperture 32 is provided in the movable support structure 30' and surface 31'. The aperture 32 is throughgoing the surface 31'. The aperture 32 allows a portion of the ultrasound probe 40 to pass through the aperture 32 so as to come into contact with the body part 41.

The movable support structure 30' is made from a material with a different impedance than that of the body part BP, such as a material comprising or consisting of a polymer and/or a metal. In other embodiments, the movable support structure 30' may be made from a material providing an impedance match, such as described with respect to movable support structure 30.

The movable support structure 30' is rotatable in the rotation direction R. In other embodiments, the movable support structure may additionally or alternatively be rotatable in the second and/or third rotation direction and/or may be translationally movable as described with respect to the movable support structure 30. Correspondingly, it will be appreciated that any feature described with respect to movable support structure 30 may apply to movable support structure 30'.

As shown in FIG. 3, the robotic system 1' further comprises a structure 41 configured to hold the ultrasound probe 40. The structure 41 is controllable by a second positioning device (not shown). The structure 41 is configured to move the ultrasound probe 40 translationally in the vertical direction V. Thereby the ultrasound probe 40 can be moved through the aperture 32 in the surface 31' of the movable support structure 30'. Specifically, the body part BP may be aligned so that the body part BP is arranged to cover the aperture 32 on one side and positioned by means of movable support structure 30 and positioning device 20 so that the aperture is arranged directly above the probe in a movement direction, i.e. the vertical direction V, of the ultrasound probe 40 and/or structure 41.

In other embodiments, the structure 41 may be controllable by the positioning device 20 and/or may be configured to move the ultrasound probe 40 translationally in the longitudinal L and/or translational direction T. Alternatively or additionally, the structure 41 may be configured to rotate the ultrasound probe 40 in rotational direction R and/or in the second and/or third rotational directions.

The structure 41 is a rigid structure in rigid connection with the ultrasound probe 40.

It will be appreciated that any feature described with respect to the robotic system 1 may equally apply to the robotic system 1'.

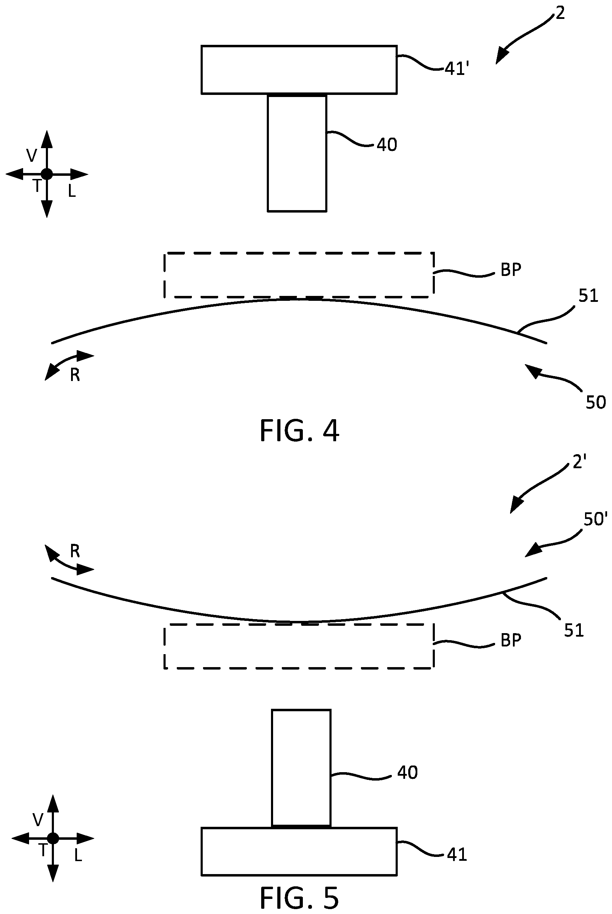

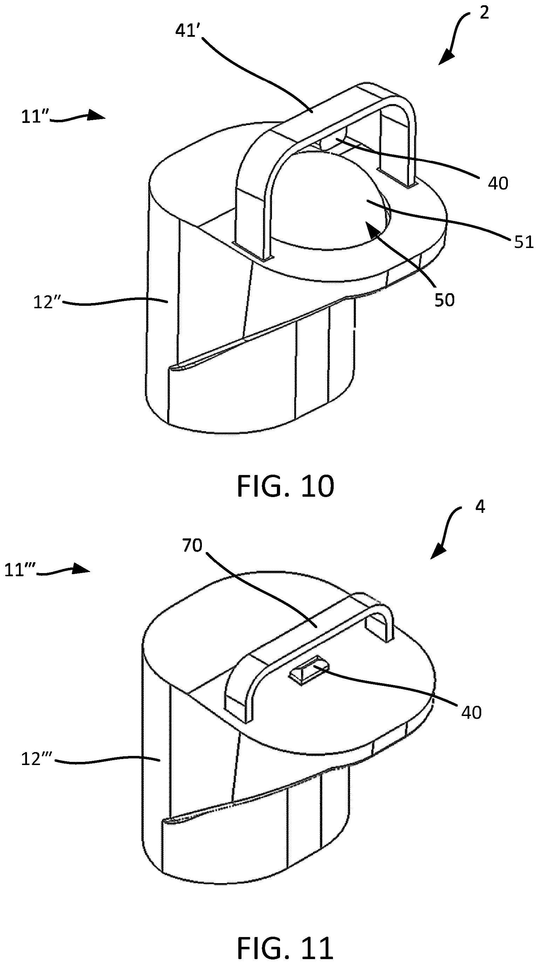

FIG. 4 shows a schematic diagram of a robotic system 2 according to an embodiment of the present disclosure. FIG. 10 shows a schematic perspective view of an ultrasound scanner 11" comprising the robotic system 2.

The robotic system 2 similar to the robotic systems 1 and 1' comprises the control unit 10 and positioning device 20.

The robotic system 2 further comprises a movable support structure 50 having a surface 51. The surface, like surfaces 31 and 31' are curved and configured to support the body part BP.

In the robotic system 2, the movable support structure 50 is configured to be arranged on an opposite side of the body part BP than the ultrasound probe 40. The movable support structure 50, like movable support structure 30', may not be made from a material, which is configured to transmit ultrasound and/or to match impedance of the body part BP. In other embodiments, the movable support structure 50 may be made from a similar or identical material to the movable support structure 30. Like movable support structures 30, 30', the movable support structure is rotatable in rotation direction R.

It will, thus, be appreciated that any feature described in relation to movable support structures 30, 30' may similarly apply to movable support structure 50, such as but not limited to the rotational and/or translational movement thereof.

The robotic system 2 further comprises structure 41' for holding the ultrasound probe 40. The structure 41' is identical to structure 41 but is, like the ultrasound probe 40, arranged on the opposite side of the body part BP than the movable support structure 50. The structure 41 is configured to move the probe in the vertical direction V, to bring the ultrasound probe 40 in an imaging position of the ultrasound probe 40, in which the ultrasound probe 40 can obtain an ultrasound scan of the body part BP. In the imaging position, the ultrasound probe 40 may be in contact with the body part BP. Correspondingly, any features described with respect to structure 41 may similarly apply to structure 41' including but not limited to movement and/or movability thereof.

FIG. 10 shows a schematic perspective view of an ultrasound scanner 11" comprising the robotic system 2. The ultrasound scanner 11" differs from ultrasound scanner 11 and 11' by comprising the robotic system 2 rather than the robotic system 1.

Correspondingly, the housing 12", contrary to housing 12 and 12' comprises mounting portions for the structure 41'. It will, however, be appreciated that any feature described with respect to ultrasound scanners 11 or 11' may similarly apply to ultrasound scanner 11", such as but not limited to materials, display unit and/or user input device. Correspondingly, any further feature described with respect to robotic systems 1 or 1' may similarly apply to robotic system 2.

FIG. 5 shows a schematic diagram of a robotic system 2' according to an embodiment of the present disclosure.

The robotic system 2' differs from robotic system 2 by comprising movable support structure 50'. The movable support structure 50', like movable support structure 50, comprises a surface 51. In robotic system 2', however, the movable support surface is arranged above the body part BP and the ultrasound probe 40 and structure 41 in the vertical direction V, contrary to the movable support structure 50 of the robotic system 2. In the robotic system 2', the movable support structure 50' is rotationally movable in rotational direction R and may be made from a non-ultrasound transmitting material or from an ultrasound transmitting material. In other embodiments, the movable support structure 50' may be rotationally movable in the second and/or third rotational direction and/or may be translationally movable in one or more of the longitudinal L, vertical V, and/or translational direction T.

Correspondingly, it will be appreciated that any feature described with respect to the robotic system 2 may similarly apply to the robotic system 2'.

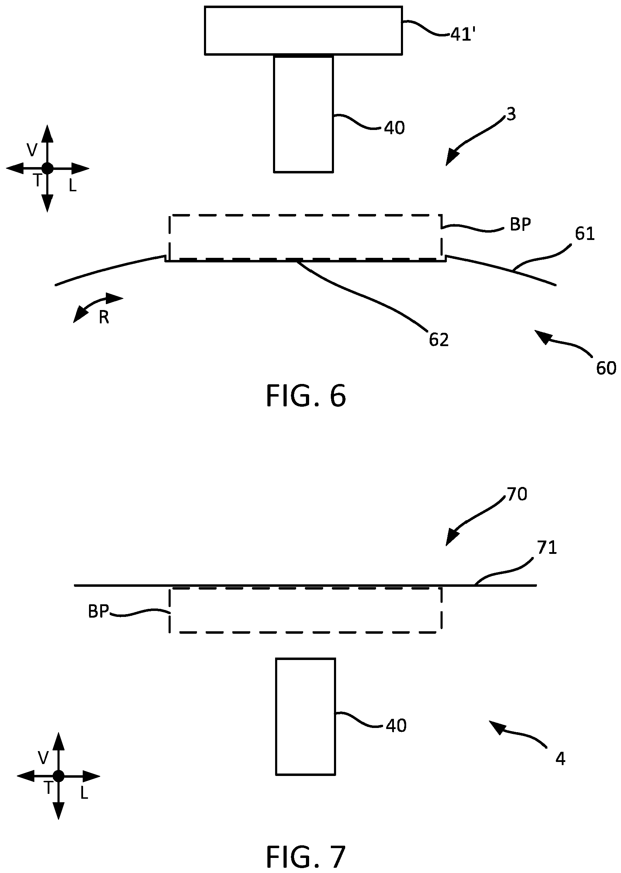

FIG. 6 shows a schematic diagram of a robotic system 3 according to an embodiment of the present disclosure.

The robotic system 3 differs from robotic systems 1, 1', 2, 2' in that the robotic system 3 comprises movable support structure 60 comprising an indentation 62 in a curved surface 61 thereof. The curved surface 61 and support structure 60 may, further comprise any same or identical feature as described with respect to movable support structures 30, 30', 50, 50', such as movement, movability, materials, and/or arrangement thereof. Like the support structures 50, 50' of robotic systems 2 and 2', the movable support structure 60 is configured to be arranged on an opposite side of the body part BP than the ultrasound probe 40. In other embodiments, however, the movable support structure 60, like support structures 30, 30' of robotic systems 1 and 1', the movable support structure 60 may be configured to be arranged on the same side of the body part BP as the ultrasound probe 40.

In the robotic system 3, the movable support structure 60 may be made from a non-ultrasound transmitting material or from an ultrasound transmitting material.

The indentation 62 conforms to the shape of the body part BP. The indentation 62 may be pre-shaped to a shape of the body part BP and/or may conform to the shape of the body part BP when the body part is arranged at and supported by the movable support structure 60.

It will be appreciated that any feature described with respect to any of robotic systems 1, 1', 2, 2' may correspondingly apply to robotic system 3.

FIG. 7 shows a schematic diagram of a robotic system 4 according to an embodiment of the present disclosure. FIG. 11 shows a schematic perspective view of an ultrasound scanner 11‴ comprising the robotic system 4.

The robotic system 4 differs from robotic systems 1, 1', 2, 2', 3 in that the robotic system 4 comprises movable support structure 70 comprising an planar surface 71. The planar surface 71 is a support surface configured to support the body part BP. Like the movable support structure 50' and surface 51 in robotic system 2', the movable support structure 70 with planar surface 71 is configured to be arranged above the body part in the vertical direction V and on an opposite of the body part BP than the ultrasound probe 40. In other embodiments, the movable support structure 70 may be configured to be arranged on the same side of the body part BP as the ultrasound probe 70, such as above or below the body part BP in the vertical direction V.

In the robotic system 3, the movable support structure 70 may be made from a non-ultrasound transmitting material or from an ultrasound transmitting material.

The movable support structure 70 of the robotic system 4 is movable translationally in the longitudinal L, vertical V, and translational direction T. In some embodiments, the movable support structure 70 may be movable translationally in one ortwo of the three directions L, V, T. In some embodiments, the movable support structure may alternatively or additionally be rotationally movable in the first, second and/or third rotation directions.

In the ultrasound scanner 11‴ the probe 40 is configured to be stationary. In other embodiments, the robotic system 4 and/or the ultrasound scanner may comprise a structure 41, 41, for holding and/or moving the probe translationally in the vertical V, longitudinal L, and/or translational direction T.

The planar surface 71 and support structure 70 may, further comprise any same or identical feature as described with respect to movable support structures 30, 30', 50, 50', 60 such as movement, movability, materials, and/or arrangement thereof. Like the support structures 50, 50', 60 of robotic systems 2, 2', and 3, respectively, the movable support structure 70 is configured to be arranged on an opposite side of the body part BP than the ultrasound probe 40. In other embodiments, however, the movable support structure 70, like support structures 30, 30' of robotic systems 1 and 1', the movable support structure 70 may be configured to be arranged on the same side of the body part BP as the ultrasound probe 40.

It will be appreciated that any feature described with respect to any of robotic systems 1, 1', 2, 2' may correspondingly apply to robotic system 3.

The ultrasound scanner 11‴ comprises a housing 12‴. The housing 12‴ differs from the housings 12, 12', 12", in that the housing 12‴ is configured to house the stationary probe in a, to a user, visible position, i.e., in a position, in which it is not hidden by a support structure of the robotic system.

Correspondingly, it will be appreciated that any further feature described with respect to the housings 12, 12', 12" may similarly apply to the housing 12‴ of the ultrasound scanner 11‴.

Additionally, variations to the disclosed embodiments can be understood and effected by the skilled person in practicing the claimed subject-matter from a study of the drawings, the disclosure and the appended claims. In the appended claims, the word "comprising" does not exclude other elements or steps, and the indefinite article "a" or "an" does not exclude a plurality. The mere fact that certain measures are recited in mutually different dependent claims does not indicate that a combination of these measures cannot be used to an advantage.

## Claims

1. A robotic system for performing an ultrasound scan on a body part of a patient using an ultrasound probe, the robotic system comprising:
a movable support structure for supporting the body part;
a positioning device configured to move the movable support structure to align the body part with the ultrasound probe to obtain an ultrasound scan of the body part supported by the movable support structure; and
a control unit configured to:
generate a first movement instruction for the positioning device for moving the movable support structure to obtain an ultrasound image of the body part.

2. The robotic system according to claim 1, where the body part is at least a part of a hand or at least part of a foot.

3. The robotic system according to any one of the preceding claims, wherein the movable support structure comprises substantially a planar surface.

4. The robotic system according to any one of the preceding claims, wherein the movable support structure comprises a substantially curved surface such as a dome shape.

5. The robotic system according to any one of the preceding claims, wherein the movable support structure has one or more indentations configured to conform to the body part.

6. The robotic system according to any of the preceding claims, wherein the movable support structure is movable translationally in one or more directions and/or rotationally around one or more axes.

7. The robotic system according to any one of the preceding claims, wherein the control unit is configured to generate a second movement instruction for moving the ultrasound probe towards the body part by means of a positioning device configured to move the ultrasound probe to obtain an ultrasound scan of the body part.

8. The robotic system according to any one of the preceding claims, wherein the movable support structure is arranged on the same side of the body part as the ultrasound probe.

9. A robotic system according to claim 8, wherein the movable support structure is configured to provide an acoustic impedance match between the body part and the ultrasound probe.

10. The robotic system according to claim 8, wherein the movable support structure has at least one aperture allowing at least a portion of the ultrasound probe to pass through the at least one aperture.

11. The robotic system according to any one of the preceding claims, wherein the movable support structure comprises fixating means for maintaining a fixed position of the body part on the movable support structure during movement of the moveable support structure.

12. An ultrasound scanner comprising the robotic system according to any one of the preceding claims and an ultrasound probe.

13. The ultrasound scanner according to claim 12 further comprising a display unit and/or a user input device.
